(19) Europäisches Patentamt
European Patent Office
Office européen des brevets

(11) EP 2 064 984 B1

(12) EUROPEAN PATENT SPECIFICATION

(45) Date of publication and mention
of the grant of the patent:
**29.06.2011 Bulletin 2011/26**

(51) Int Cl.:
*A61B 1/018* (2006.01)    *A61B 17/28* (2006.01)

(21) Application number: **08018968.1**

(22) Date of filing: **30.10.2008**

(54) **Therapeutic device system and manipulator system**

Therapievorrichtungssystem und Manipulatorsystem

Système de dispositif thérapeutique et système de manipulateur

(84) Designated Contracting States:
**DE GB**

(30) Priority: **29.11.2007 JP 2007308830**

(43) Date of publication of application:
**03.06.2009 Bulletin 2009/23**

(60) Divisional application:
**10004087.2 / 2 213 221**

(73) Proprietor: **Olympus Medical Systems Corp.**
**Tokyo 151-0072 (JP)**

(72) Inventors:
• **Takahashi, Kazuhiko**
**Tokyo 192-8512 (JP)**
• **Nakamura, Toshio**
**Tokyo 192-8512 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
WO-A-99/33392    WO-A-2007/111571
DE-A- 3 734 979

## Description

The present invention relates to a therapeutic device system and a manipulator system including an active therapeutic device to be inserted into an insertion section of an endoscope apparatus.

In general, a manipulator in which a plurality of rods are universal-joint-connected to each other by means of articulation sections, and which can be freely curved is known as one of the master-slave type arm robot. For example, there is a manipulator used as a therapeutic device which is inserted into a therapeutic device hole of an endoscope insertion section of an endoscope apparatus to be used for various treatments.

For example, document WO 2007/111571 A1 describes a surgical robotic system for flexible endoscopy. According to one exemplary embodiment, a slave robotic manipulator has six degrees of freedom and is anthropomorphic to the human arm. Two slave manipulators are attached to the distal end of a conventional flexible endoscope using an attachment that attaches to the distal end and supports the manipulators. The manipulators are actuated by tendon-sheath-cables connected to motors at the proximal ends.

Document DE 37 34 979 A1 describes an Endoscope. This endoscope is provided with an operating section and an insertion section having a bending section. Bending members, which are formed of a shape memorizable alloy are disposed within the bending section of the insertion section and are connected to a power supply unit. This endoscope further comprises a detecting unit for detecting the amount of deformation of the bending members as a change in electric resistance, and a display device for displaying of the bending members. Accordingly, the bending amount of the bending section can accurately be detected and displayed.

Document WO 99/33392 A1 describes a deformable probe with automatic detection of the position of the probe. The probe comprises a plurality of deformation and distortion sensors which are distributed along the length of the probe. The supply lines are preferably guided outward in a multiplex circuit and are connected to a computer. A differential iterative computing method is used for calculating the deformation and position of the probe, and the position of the probe is displayed on a screen.

Various therapeutic devices corresponding to uses such as an eledric-cautery, forceps, and the like are attached to the distal end section of the manipulator.

The inside of each of the rods of this manipulator is connected to a drive wire, and the articulation section is bent by pulling the wire. When the wire pulling operation is performed, at least two wires are used for one articulation section, i.e., one degree of freedom, and hence twice as many wires as the number of articulation sections are arranged. By adjusting the traction amount of each of the wires, the positional posture can be changed as desired.

Normally, the wires of the manipulator are arranged in the therapeutic device insertion section.

In the therapeutic device insertion section, wires which are inserted into the endoscope insertion section, and are led to the proximal end side of the endoscope are connected to the actuator serving as the drive section.

This manipulator is on the slave side, and performs an operation in accordance with the manipulation of the manipulation section of the master side arranged outside.

In general, a curving operation of the manipulator has an operation amount corresponding to a manipulation amount of the manipulation section. However, as a system configured in consideration of the individual character of the operator, there is, for example, a system equipped with an electric curving mechanism described in Jpn. Pat. Appln. KOKAI Publication No. 8-071072.

In this system, a drive signal is generated by adjusting an actual manipulation amount of the manipulation section by using a fixed control parameter fitted to the individual character of the operator, and an electric curving operation conformed to the drive signal is realized. That is, a movement amount and a moving speed of the manipulator is controlled in consideration of the personal habit and the degree of proficiency of the operator, thereby realizing further improvement in the degree of safety and the operability.

When the manipulator of the therapeutic device is used for a soft endoscope apparatus, it is used for an insertion part having flexibility and inserted into the therapeutic device hole of the insertion section of the endoscope, and a curving section of the multiarticular structure having a high degree of freedom provided on the distal end side. In the curving section of the multiarticular structure, the articulation section is curved by the traction of the wires.

Accordingly, the positional posture of an end effector (for example, a therapeutic device) provided at the distal end of the curving section are determined by the articulation parameter (in this case, an angle formed between articulations) of each articulation section.

Accordingly, an inverse problem for obtaining a target value of the articulation parameter for coinciding the positional posture of the end effector with the target positional posture of the operator is solved and, thereafter, drive control is performed in such a manner that the current articulation parameter value coincides with the target parameter value. As described above, in the therapeutic device, the insertion section and the curving section are curved in accordance with the shape inside the body cavity of the patient, and hence the operation is performed in consideration of the curved state of the insertion section.

An embodiment according to the present invention provides a therapeutic device system and a manipulator system which detect a curved state of an endoscope insertion section, adjust an operation of a therapeutic device inserted into the insertion section and an operation of a manipulator in accordance with the curved state, and operate smoothly with excellent operability.

Further, an embodiment according to the present invention provides a therapeutic device system comprising: an input section for generating a manipulation signal from a manipulation-designated amount designated by manipulation section; any one of a therapeutic device and a manipulator which includes articulation sections, a therapeutic device insertion section of which is inserted into a therapeutic device hole formed through to any one of an endoscope insertion section and an overtube attached to the outside of an endoscope, and in which the articulation sections can perform an articular operation in accordance with a manipulation instruction of the input section; a detection section for detecting a curved state of the curving section at all times; a therapeutic device drive section for driving the therapeutic device in accordance with the manipulation signal; and a control section provided with a control parameter set in advance, for controlling the articular operation of the articulation sections by changing the control parameter at all times on the basis of a detection result from the detection section, and causing the therapeutic device drive section to make the manipulation signal reflect the changed control parameter.

The invention can be more fully understood from the following detailed description when taken in conjunction with the accompanying drawings, in which:

FIG. 1 is a block diagram showing the configuration of a therapeutic device system.
FIGS. 2A and 2B are views each showing the specific configuration of a multiarticular manipulator of this embodiment.
FIG. 3 is a view showing an example of a multiarticular structure model of the manipulator of this embodiment.

An embodiment of the present invention will be described below in detail with reference to the accompanying drawings.

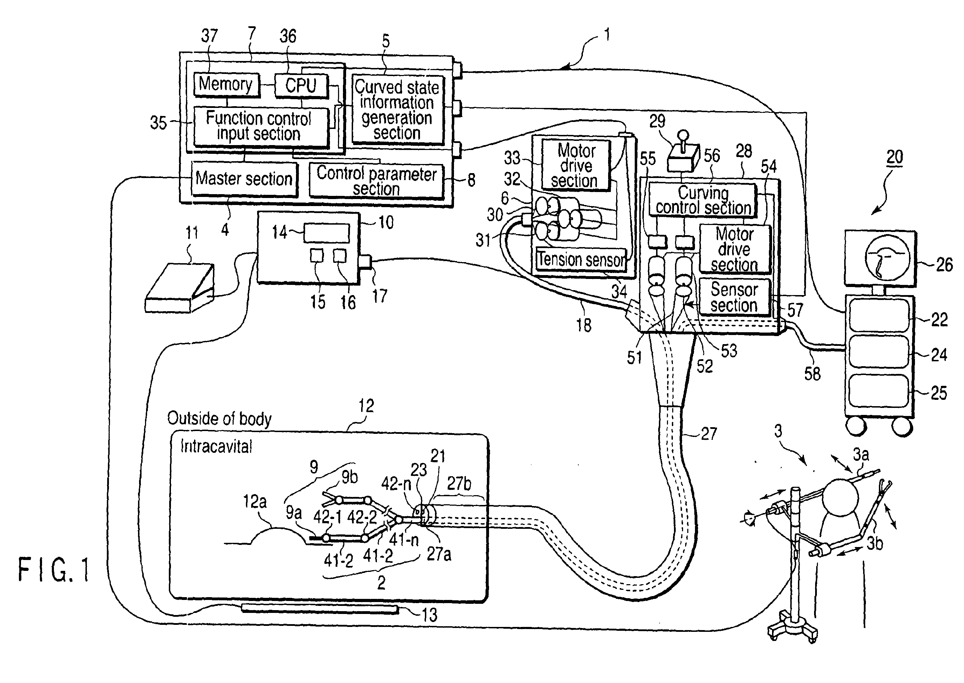

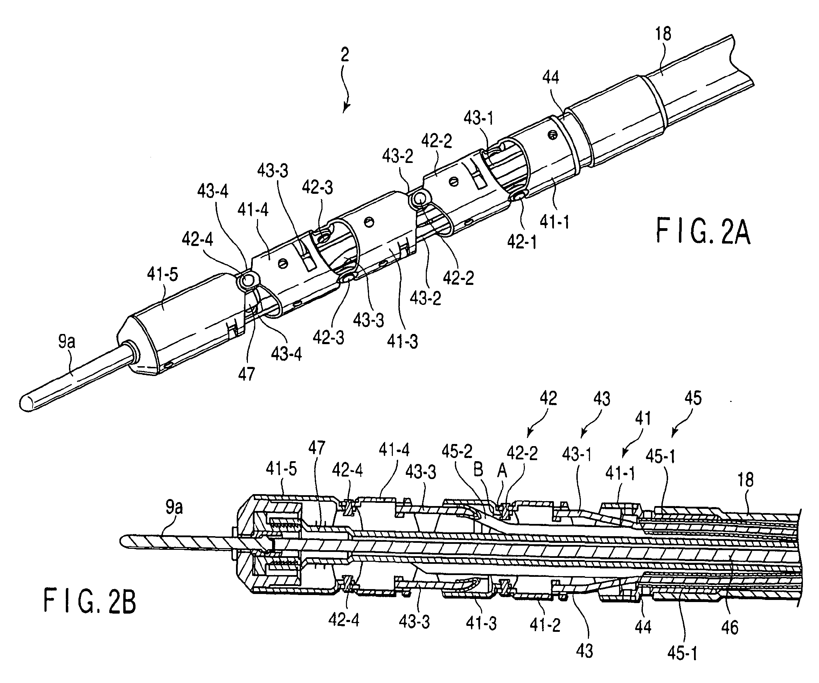

A therapeutic device system and a manipulator system of a first embodiment according to the present invention will be described below. FIG. 1 is a block diagram showing the configuration of the therapeutic device system. FIGS. 2A and 2B are views each showing the specific configuration of a multiarticular manipulator 2 used in the therapeutic device or the like of this embodiment.

In the therapeutic device system of this embodiment, a therapeutic device and/or a manipulator inserted into an insertion section of an endoscope apparatus generate or generates, with respect to a change in force exerted in a state where the insertion section of the endoscope apparatus is curved, a control parameter corresponding to the curved state, and the control parameter is added to a drive signal for driving the therapeutic device and the manipulator, whereby an articular operation is performed at a position or an angle desired by the operator.

The therapeutic device system 1 is inserted into a therapeutic device hole (forceps channel hole) of an endoscope insertion section 27 of an endoscope apparatus 20, or a therapeutic device hole of an overtube attached to the outside of the endoscope. The therapeutic device system 1 is a master-slave type electric therapeutic device for causing a therapeutic device and a manipulator extending from a distal end a therapeutic device hole thereof to perform a curving operation and a treating operation by means of, for example, an articulation section and wire traction.

Incidentally, it is assumed that endoscopes and therapeutic devices described in the following embodiments are active endoscopes and active therapeutic devices for performing operations such as a curving operation of the articulation section, and operations such as opening/closing, grasping, and the like at the movable part of the therapeutic device by means of a power source such as a motor and the like. Further, the above endoscopes and therapeutic devices are simply called endoscopes and therapeutic devices in the following description. Further, in addition to the electric drive source for driving by the magnetic force such as that of a motor or the like, a hydraulic drive source, and a pneumatic pressure drive source also belong to the category of the power source.

This therapeutic device system 1 is roughly comprised of a therapeutic device insertion section 18 which is inserted into a therapeutic device hole of an endoscope insertion section 27, can be advanced or retreated, and can be freely curved, a manipulator 2 provided at a distal end of the therapeutic device insertion section 18, and extending outwardly from a therapeutic device hole opening 27a of the endoscope insertion section 27, a manipulation section 3 by which the operator performs an operation instruction, a master section 4 for generating a manipulation signal corresponding to a manipulation amount of the manipulation section 3, a curved state information generation section 5 for generating curved state information to be described later, a manipulator drive section (actuator) 6 for driving the manipulator 2, a therapeutic device control section 7 for generating a control signal by adjusting the manipulation signal from the master section 4 by using a control parameter, and drive-controlling the manipulator drive section 6, and a control parameter section 8 for calculating a control parameter for adjusting the drive amount of the manipulator with respect to the manipulation signal on the basis of the curved state information, and providing the calculated control parameter to the therapeutic device control section 7.

Incidentally, in this embodiment, a curving operation of the articulation section of each of the therapeutic device and the manipulator inserted into the soft endoscope insertion section will be described below as an example. However, in these articulation sections, some of them perform not only a curving operation, but also, for example, an opening/closing operation of a linear-motion therapeutic device. The curving operation can also be easily applied to these articulation sections.

Incidentally, in this embodiment, the master section 4, curved state information generation section 5, therapeutic device control section 7, and control parameter section 8 are contained in a housing, and function as a control section of the therapeutic device system 1. Further, in the following embodiment, an articular operation of the manipulator 2 will be mainly described as an example.

In this embodiment, an example in which an electric-cautery 9a and a grasping instrument (for example, a forceps) 9b are provided as a therapeutic device unit is shown. Further, the electric-cautery 9a is used, and hence a power supply device 10 for supplying high-frequency power to the electric-cautery 9a, a foot switch 11 for issuing an instruction to supply high-frequency power to the high-frequency electric-cautery by a foot operation of the operator, and a counter electrode plate 13 connected to the power supply device 10, and attached to a body surface of a patient 12 to be subjected to a treatment are further provided. Incidentally, as for the therapeutic device, general therapeutic devices, or a combination of these may be provided in addition to the electric-cautery 9a and the forceps, and the like.

The power supply device 10 is provided with a display 14 for displaying a supply state and the like of power, an output wattage input panel 15, an output mode selection panel 16, and a power output terminal 17. The power output terminal 17 supplies high-frequency power output from a power unit (not shown) provided inside to the electric-cautery 9a.

The endoscope apparatus 20 is constituted of an image processing section 22 for subjecting image data taken by an image pickup section 21 provided at a distal end of the endoscope insertion section 27 to various image processing and data processing, a light source section 24 arranged adjacent to the image pickup section 21, for generating illumination light for illuminating an observation visual field including a lesioned part 12a from an illumination light window 23 through a light guide fiber (not shown), an endoscope control section 25 for performing control of the entire endoscope apparatus system, arithmetic processing, and the like, a monitor 26 for displaying a taken image, data relating to the image, an apparatus state, a manipulation instruction, and the like, an endoscope insertion section 27 a distal end part of which is provided with a multiarticular mechanism equivalent to the manipulator 2, and which is provided with an endoscope curving section 27b that can be curved, an electric-powered curving manipulation section 28 for curving the endoscope curving section 27b by means of electric power, and a curving joystick 29 for instructing to perform a curving manipulation of the endoscope.

The electric-powered curving manipulation section 28 has a configuration substantially equivalent to the manipulator drive section 6 to be described later, and includes a plurality of wires 51 for traction, a plurality of pulleys 52 each of which is connected to the other end of each wire 51, motors 53 rotation shafts of which are each fitted with the pulleys 52, a motor drive section 54 for individually driving each motor 53, encoders 55 with which the motor 53 are provided, a curving control section 56 for controlling the motor drive section 54 on the basis of values detected by the encoders 55, and a sensor section 57 including tension sensors for detecting values of tension exerted on the wires 51, and a strain gauge for detecting a strain amount of the endoscope insertion section 27. Further, the curving control section 56 is connected to the curving joystick 29, and an instruction of the curving manipulation is input thereto. Further, the curved state information generation section 5 generates curved state information from pulley angular information (wire traction information) of the pulley 52 detected by the encoder 55 functioning as a sensor section.

Further, the electric-powered curving manipulation section 28 is connected to the apparatus main body 20 with a cable 58. This cable includes the light guide fiber for sending illumination light, and signal lines constituted of an image signal line, a control signal line, and the like. Further, in this embodiment, the configuration example in which each of the endoscope and the therapeutic device is provided with a joystick is shown. However, the configuration may be made in such a manner that these manipulation functions are integrated into one joystick. Further, the example in which the therapeutic device system of this embodiment is applied to the endoscope apparatus configured in such a manner that the electric-powered curving manipulation section 28 and the endoscope insertion section 27 are fixedly connected to each other is described. However, it is possible to also apply the therapeutic device system of this embodiment to an endoscope apparatus so configured as to allow the endoscope insertion section to be attachable/detachable to/from the electric-powered curving manipulation section. Incidentally, in the case of a configuration in which a plurality of endoscope insertion sections can be selectively connected to one electric-powered curving manipulation section in turn, it is sufficient if a specific control parameter is registered in advance in a table of a memory provided in the control section for each endoscope insertion section, and a control parameter corresponding to the endoscope insertion section is read and set when the endoscope insertion section is connected to the electric-powered curving manipulation section. Further, it is desirable that a sensor and the like for detecting the curved state of the endoscope insertion section be provided as in this embodiment.

The therapeutic device system of this embodiment will be described below in detail.

First, the configuration of the manipulator 2 of the embodiment will be described below. FIG. 2A shows an example of the external appearance configuration of the manipulator 2, and FIG. 2B shows an example of the cross-sectional configuration of the manipulator 2.

This manipulator 2 includes a plurality of cylindrical curving pieces 41 (41-1, 41-2, 41-3, 41-4, and 41-5), axle members 42 (42-1, 42-2, 42-3, and 42-4) for coupling these curving pieces 41 so that they can be freely bent and curved, an electric-cautery 9a (or a grasping section 9b) provided on the curving piece 41-1 on the distal end side of the manipulator 2, a therapeutic device insertion section 18 which can be bent or curved in a comparatively soft and elastic manner, and a coupling member 44 for coupling the therapeutic device insertion section 18 and the curving piece 41-1 on the proximal end side to each other.

The inside of the linked structure of these curving pieces 41 and the axle members 42 is constituted of wires 43 (43-1, 43-2, 43-3, and 43-4) each of which is fixed to each of the curving pieces 41 at a distal end part by brazing or the like, and in which at least two wires are paired, flexible coils 45 (45-1, 45-2, 45-3, and 45-4) through which the wires are respectively passed, and which are provided between the respective curving pieces 41 and the connector of the actuator 6 in a penetrating manner, a power supply line 46 for supplying high-frequency power to the electric-cautery 9a, and a flexible tube provided in such a manner that the power supply line 46 is passed therethrough from the proximal end side of the endoscope insertion section 27 to the curving piece 41-5 at the distal end of the manipulator 2.

The linked structure of the curving pieces 41 and the axle members 42 will be described below.

Each of the curving pieces 41 excluding the curving pieces arranged at the distal end and the trunk end (proximal end side) is provided with two distal end side protrusion sections each having a tongue-like shape provided on the distal end side thereof with the central axis of the cylindrical curving piece interposed between them, and two tongue-like proximal end side protrusion sections provided on the proximal end side thereof with the central axis of the cylindrical curving piece interposed between them in a direction perpendicular (90° rotation) to the distal end side protrusion sections.

The linkage configuration between adjacent curving pieces 41 is such that as shown in FIG. 2A, for example, a hole is formed in each of the proximal end side protrusion section A of the curving piece 41-2, and the distal end side protrusion section B of the curving piece 41-3, the holes are caused to overlap each other, and the rivet-like axle member 42-2 is inserted into the holes, and thus the curving pieces 41-2 and 41-3 are linked with each other so that they can be swung, i.e., they can be curved freely. In this manner, the curving pieces 41 are universal-joint-connected to each other by means of the axle members 42, thereby forming a plurality of linked stages. By the linkage described above, in the linkage structure, each curving piece 41 is shifted 90° from the curving pieces adjacent in front and behind in posture.

In the universal-joint-connected linkage structure in which the connection positions of the axle members 42 between the adjacent curving pieces 41 are alternately shifted 90° as described above, by pulling one of the wires 43 of a desired curving piece 41, the curving piece is swung around two axle members 42. Accordingly, it is possible to perform a so-called articular operation in which two curving pieces 41 are freely curved or stretched straight according to the degree of the traction of the wire 43 of the desired curving piece 41, and move the electric-cautery 9a or the grasping section 9b three-dimensionally to a desired position.

The manipulator drive section 6 drives the manipulator 2 by means of electric power in accordance with a control signal from the therapeutic device control section 7. The manipulator drive section 6 is constituted of a plurality of wires 30 each of which has one end connected to a curving piece 41 of the manipulator 2, a plurality of pulleys 31 each of which is connected to the other end of each of the wires 30, motors 32 each of which serves as the drive source of the wire traction, rotation shafts of which are each fitted with the pulleys 31, a motor drive section 33 for individually driving each motor 32, and tension sensors 34 for detecting tension of the wires 30.

The manipulation section 3 is constituted of arm manipulation sections 3a and 3b each having a simplified multiarticular arm mechanism as shown in FIG. 1. As a sensor for detecting the arm manipulation, a magnetic sensor or an acceleration sensor is used, and an operation amount and an operation direction of each curving piece of the arm mechanism are detected. As the other sensor, a light emission source (for example, a laser light emitting element) is provided in each curving piece, and a light receiving element is arranged on a fixed member such as a support. It is also possible to detect the operation amount and the movement direction by an incident angle, signal strength (degree of attenuation), and the like of light incident on the light receiving element.

A manipulation signal based on a movement amount (manipulation instruction amount) of the arm manipulation sections 3a and 3b manipulated by the user is generated by the master section 4, and is output to the therapeutic device control section 7. In the manipulation section 3, as general input parts other than the arm mechanism, for example, a button switch, a joystick, a keyboard, and the like can be used. The manipulation instruction issued from the manipulation section 3 has a master-servant relationship with respect to the manipulator 2, and the manipulator 2 serving as the servant executes a curving operation or treatment in accordance with the manipulation instruction issued from the manipulation section 3 which is the master. Further, the manipulator 2 is inserted into the body cavity, and is remotely controlled, and hence the therapeutic device cannot be visually confirmed in a direct manner. Accordingly, the manipulation section 3 is manipulated while a dynamic picture image taken by the endoscope is viewed on the monitor 26, thereby sending an instruction from the master section 4.

As for the curved state of manipulator 2 of the therapeutic device, the positional posture thereof can be found from the tension value of the tension sensor 34. Here, the curving operation of the manipulator 2 will be described below.

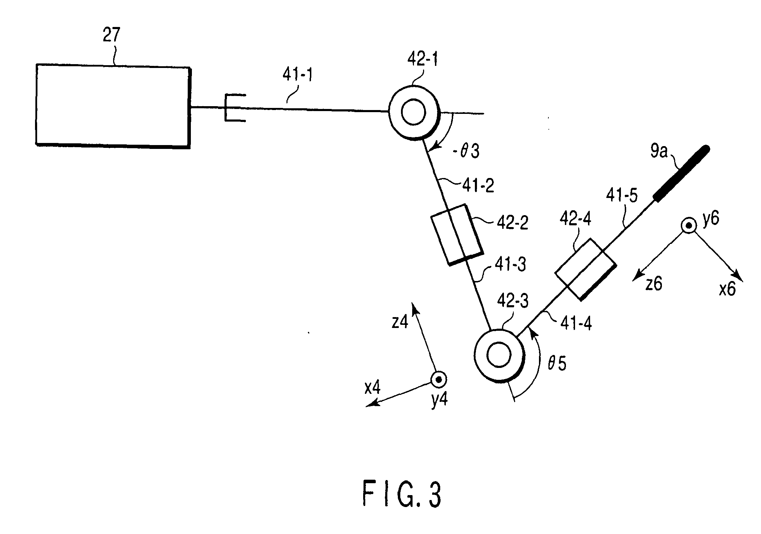

FIG. 3 shows a multiarticular structure model having four degrees of freedom provided by the five curving pieces 41-1 to 41-5, and the four articulation sections 42-1 to 42-4 of the manipulator 2 of this embodiment. Incidentally, although not shown in FIG. 3, on the proximal end side of a normal manipulator 2, axle members for curving the entire manipulator 2 in the axial direction, and axle members for moving the entire manipulator 2 in the direction around the axis are provided. The four axle members 42-1 to 42-4 curve the curving pieces alternately in the axial direction and in the direction around the axis. In the configuration described above, in, for example, FIG. 3, the state where both the axle member 42-1 and the axle member 42-3 are turned, and the part between the curving piece 41-1 and the curving piece 41-2, and the part between the curving piece 41-3 and the curving piece 41-4 are bent is shown. The current positional posture of the multiarticular manipulator 2 is detected. When the articulation angle (an articulation parameter which is one of so-called control parameters) of the axle members 18 is expressed by the following expression (1),

$$\Phi = \left( \theta_1, \theta_2, \cdots, \theta_n \right)^T \tag{1}$$

as shown in, for example, FIG. 3, the folding angle of the axle member 42-1 becomes $-\theta 3$, and the folding angle of the axle member 42-3 becomes $\theta 5$. As for the folding angles of the axle members 42 other than these, the articulation parameters remain 0 as long as they do not change from the initial positions. The positional posture of the electric-cautery 9a can be expressed by the following expression (2) as follows.

$$E_p = \left( x_{Ep}, y_{Ep}, z_{Ep}, Roll_{Ep}, Yaw_{Ep}, Pitch_{Ep} \right)^T \tag{2}$$

The relationship can be expressed by the following expression (3) as follows.

$$E_p = A(\Phi) \tag{3}$$

Here, the current positional posture of the electric-cautery 9a is expressed by the following expression (4) as follows.

$$E_{pnow} = \left( x_{Epnow}, y_{Epnow}, z_{Epnow}, Roll_{Epnow}, Yaw_{Epnow}, Pitch_{Epnow} \right)^T \tag{4}$$

The target positional posture to which the electric-cautery 9a is moved is set by the following expression (5) as follows.

$$P_p = \left( x_{Pp}, y_{Pp}, z_{Pp}, Roll_{Pp}, Yaw_{Pp}, Pitch_{Pp} \right)^T \tag{5}$$

Then, in order to bring the electric-cautery 9a into the state of the target position Pp, it is necessary to change the articulation parameter $\Phi$ from $\Phi$ satisfying the following expression (6)

$$E_{pnow} = A(\Phi_{now}) \tag{6}$$

to $\Phi$ satisfying the following expression (7).

$$P_p = A(\Phi_P) \tag{7}$$

These relational expressions are nonlinear, and hence, in order to obtain Φp, the Jacobian determinant J(Φ) obtained by subjecting Ep to partial differentiation by using the element of Φ is obtained as follows.

$$J(\Phi) = \begin{pmatrix} dx_{ep}/d\theta_1 & dx_{ep}/d\theta_2 & \cdots & dx_{ep}/d\theta_n \\ dy_{ep}/d\theta_1 & dy_{ep}/d\theta_2 & \cdots & dy_{ep}/d\theta_n \\ dz_{ep}/d\theta_1 & dz_{ep}/d\theta_2 & \cdots & dz_{ep}/d\theta_n \\ dRoll_{ep}/d\theta_1 & dRoll_{ep}/d\theta_2 & \cdots & dRoll_{ep}/d\theta_n \\ dYaw_{ep}/d\theta_1 & dYaw_{ep}/d\theta_2 & \cdots & dYaw_{ep}/d\theta_n \\ dPitch_{ep}/d\theta_1 & dPitch_{ep}/d\theta_2 & \cdots & dPitch_{ep}/d\theta_n \end{pmatrix} \qquad (8)$$

From the following expression (9),

$$\dot{\Phi} = J(\Phi)^{-1} \dot{E}_p \qquad (9)$$

Φp satisfying the following expression (10)

$$P_p = A(\Phi_P) \qquad (10)$$

$$\alpha = (a_1, a_2, \cdots, a_n) \qquad (11)$$

can be obtained by convergent calculation. These calculation operations, i.e., calculation of the target positional posture is operation-processed by a CPU 36 in the therapeutic device control section 7.

The curved state information generation section 5 will be described below.

The curved state information generation section 5 generates curved state information (curved attitude position) on the endoscope insertion section 27 and the curving section 27b from the detection value of the sensor section 57.

Here, the curved state information is constituted of at least curved state information on the endoscope insertion section 27, curved state information on the curving section 27b provided on the distal end side of the endoscope insertion section 27, and the rotation amount of the pulley 52, or the wire length of the wire 51 pulled by the rotation amount of the pulley 52, or curved state information on the paid-out wire of the paid-out length.

Of these pieces of information, the curved state information on the insertion section 27 is information on the strain amount of the insertion section 27 detected by using a strain gauge of the sensor section 57. It is possible to estimate the current curved state of the insertion section 27 from this strain amount. Incidentally, it is also possible to detect a change in the curved state of the insertion section 27 by using the tension sensor of the sensor section 57. By assuming that the tension value of the tension sensor in the state where the curving section 27b is linear (i.e., in the state where no load is imposed on the curving wire of the therapeutic device) to be the initial value, it is possible to find the degree of the curved state of the curving section 27b from a change in the tension value.

Next, the control parameter section 8 will be described below.

First, the necessity of changing the control parameter will be described below.

In this embodiment, at the time of treating an affected part by using a therapeutic device, the manipulator 2 is driven to be curved by the traction of the wires performed by using the motors as drive sources, and the therapeutic device is set to a desired position of the affected part. The therapeutic device insertion section 18 is passed through the endoscope insertion section 27 and the curving section 27b which are curved in accordance with the shape inside the body cavity of the patient. Accordingly, the smaller the arc of the curvature is or the more the number of curved parts of the endoscope insertion section 27 or the curving section 27b is, the more the path length of the wires 30 arranged inside the therapeutic device insertion section 18 is changed, or the more the load is changed to be imposed.

As described previously, even when the convenience of manipulation is provided by using a control parameter (fixed value such as the articulation parameter or the like) set in advance, a situation different from the movement (the moving speed and the degree of the curved state) assumed by the operator occurs due to the load changing in accordance with the curved state.

In this embodiment, the control parameter is changed each time the change amount (signal value) of the curved information determined in advance is exceeded. That is, each time a certain change in the curved state occurs, the control parameter is changed (rewritten) in accordance with the curved state in the insertion section and the curving section, whereby even when the curved state is changed, a movement (the moving speed or the degree of curvature) of the manipulator or the therapeutic device conforming to the manipulation of the operator is performed. Incidentally, changing the control parameter may be performed not only in accordance with the change amount of the curved state but also on the basis of a predetermined period of time. The changing of the control parameter is continuously performed as long as the therapeutic device or the manipulator 2 is driven. That is, during the drive, the control parameter is changed at all times.

The control parameter section 8 of this embodiment stores therein a control parameter for adjusting the manipulation signal with respect to the drive amount of the manipulator 2 on the basis of the curved state information, concomitantly with a change in the curved state, calculates the control parameter as required in accordance with an operational expression or a program set in advance, and outputs the calculated control parameter to a corresponding table of a control table provided in the therapeutic device control section 7 as required, thereby updating the table.

The therapeutic device control section 7 is constituted of a function control input section 35 for inputting a manipulation instruction from the master section 4, and a control condition of a function or a control parameter from the control parameter section, a central processing unit (CPU) 36 for performing various operation processing, and instruction to each constituent section, and a memory 37 for storing images, communication data, and the like. The CPU 36 detects the positional posture (including the curved state) of the manipulator 2, and the operation state of the therapeutic device 9 by means of the detection signal of the tension sensor 34. In the memory 37, initial data at the operation start-up time, and an ID parameter (individual input ratio) for setting an operation condition for each selectable operator are stored. The ID parameter is a parameter for adjustment that enables standard manipulation or proper manipulation to be obtained while eliminating the personal habit of the operator in the manipulation.

The control parameters include teaching data of a slave manipulator for observation and treatment, a master-slave scale ratio, sensitivity, and the like.

Of these parameters, the master-slave scale ratio is a parameter for determining how the operation amount of the manipulator 2 should be with respect to the operation amount of the arm of the manipulation section 3. In the case where the operation amount of the arm is the movement distance of the therapeutic device, and when the master-slave scale ratio is set at, for example, 1, and the movement amount of the distal end of the manipulation section 3 is 10 mm, the manipulator 2 operates in such a manner that the movement amount of the therapeutic device becomes 10 mm. On the other hand, when the master-slave scale ratio is 0.1, and when the movement amount of the distal end of the manipulation section 3 is 10 mm, the manipulator 2 operates in such a manner that the movement amount of the therapeutic device becomes 1 mm. In this example, although the scale ratio is that for positional movement, the scale ratio also applies to the angle ratio between the master side and the slave side.

Further, in the case where the sensitivity is used as the control parameter, for example, when a magnetic sensor is attached to the arm of the manipulation section 3, and the sensor signal is used as the curved state information, by changing the input sensitivity, the width of the dead band of the manipulator 2 can be changed. For example, when the input sensitivity is set at 1 mm, the manipulator 2 does not operate as long as the magnetic sensor does not move 1 mm or more.

This makes it possible to eliminate a useless operation of the manipulator resulting from a shake or wobble of the operator's hand.

Next, an example of a change (rewriting) of the control parameter concomitant with calculation of a control parameter using curved state information, and a change in the curved state information will be described below.

For example, when the curved state of the endoscope is set as $\varepsilon$, the following are defined.

no curved state (straight, and not curved) $\varepsilon = 0$

a curved state on the positive side (upwardly curved when, for example, the horizontal direction is set as 0) $\varepsilon > 0$

a curved state on the negative side (downwardly curved when, for example, the horizontal direction is set as 0) $\varepsilon < 0$

Further, as for the lateral direction, judgment may be made likewise by setting the forward direction as 0, the right direction as the positive side, and the left side as the negative side. Needless to say, such setting may be appropriately determined at the time of setting.

Here, when there is no curved state ($\varepsilon = 0$), if the motor target angle input and set by the operator is set as $\theta1$, and the motor target angle determined by the detected curved state $\varepsilon$ is set as $\theta2$, $\theta2$ is obtained from the following function.

$$\theta2 \ = \ F(\theta1, \ \varepsilon)$$

The function $F(\theta1, \varepsilon)$ is, for example, the following.

$$F(\theta1, \ \varepsilon) \ = \ \theta1 \ + \ D\varepsilon \quad (D: \ constant)$$

As described above, according to the therapeutic device system of this embodiment, the control parameter for adjusting the relationship between the amount of manipulation made by the operator, and the operation amount of the manipulator provided with the therapeutic device is changed in accordance with the curved state of the endoscope insertion section. Even when the curved state of the endoscope insertion section is changed as a result of this change, the control parameter in the changed state is calculated, and the previous control parameter is rewritten. Accordingly, it is possible for the operator to operate the manipulator by the same manipulation operation at all times. Therefore, unlike the conventional case, the operability of the manipulator becomes better without being affected by the curved state of the endoscope insertion section, and the labor required to perform the treatment is reduced.

Next, the drive control of the therapeutic device system performed by the therapeutic device control section 7 will be described below.

First, the therapeutic device control section 7 is subjected to initialization processing at the time of turn-on and start-up. At this time, teaching data set in advance is set as the initial data. Hereine, when there is an ID parameter set in accordance with the individuality of the operator in the manipulation, the ID parameter is read from the memory file, and condition-setting is performed. At the same time, as for the proper control parameter owned by each therapeutic device insertion section 18 of the endoscope apparatus to be used, the operator inputs or registers in advance a control parameter owned by each therapeutic device to be used in a table of a memory (not shown) in the control parameter section 8, and a control parameter is selected and read from the parameter group, whereby the initial setting is performed.

The operator (operating surgeon) grasps the manipulation section 3, and performs an operation while viewing the monitor 26. In accordance with the movement of the hands of the operator, a signal indicating the manipulation-designated amount is input from the arm manipulation section 3a or 3b to the master section 4. The master section 4 generates a manipulation signal from the input signal, and outputs the manipulation signal to the function control input section 35.

Further, curved state information is generated from the curved state of the endoscope insertion section 27 detected by the sensor section 57 and the encoder 55, and is output to the function control input section 35. Likewise, a detection signal is output from the tension sensor 34 to the function control input section 35. The function control input section 35 outputs the manipulation signal, the curved state information, and the control parameter to the CPU 36. In the CPU 36, operation processing is performed by the operation method described previously, control signals based on the operation result are output to the respective motor drive sections 33 and 54, and the respective articulation sections constituted of the respective curving pieces 41 are caused to perform an articular operation (bending or linear extension), whereby the therapeutic device is moved to a position desired by the operator.

As has been described above, in the therapeutic device system of this embodiment, the therapeutic device and/or the manipulator inserted into the insertion section of the endoscope apparatus generate or generates, with respect to a change in force exerted in a state where the insertion section of the endoscope apparatus is curved, a control parameter corresponding to the curved state, and the drive signal at the therapeutic device or the manipulator is adjusted so as to adjust the operation, whereby an articular operation is performed at a position or an angle desired by the operator, and the operation can be performed with good operability and smoothly.

**Claims**

**1.** A therapeutic device system comprising:

an input section (3) for generating a manipulation signal from a manipulation-designated amount designated by manipulation sections (3a and 3b);

any one of a therapeutic device (9) and a manipulator (2) which includes articulation sections (42), a therapeutic device insertion section of which is inserted into a therapeutic device hole formed through to any one of an endoscope insertion section (27) and an overtube attached to the outside of an endoscope, and in which the articulation sections (42) can perform an articular operation in accordance with a manipulation instruction of the input section;
a therapeutic device drive section (6) for driving the therapeutic device (9) and the manipulator (2) in accordance with the manipulation signal;
**characterized by**
a detection section (57) for detecting a curved state of the endoscope insertion section (27) at all times; and
a control section (7) provided with a control parameter set in advance, for controlling the articular operation of the articulation sections by changing the control parameter at all times on the basis of a detection result from the detection section (57), and causing the therapeutic device drive section (6) to make the manipulation signal reflect the changed control parameter.

2. The therapeutic device system according to claim 1, **characterized by** further comprising, in order to cause the articulation sections (42) of the therapeutic device insertion section (27) to perform an articular operation:

   wires (43) connected to the articulation sections (42);
   pulleys (31) for performing traction of the wires (30) by rotation; and
   a pulley drive section (33) for rotating the pulleys (31).

3. The therapeutic device system according to claim 1, **characterized in that**
   the detection section (57) is constituted of one of
   a strain gauge for detecting a curve of a curving section (27b) provided at a distal end section of the endoscope insertion section, and the insertion section (27) located behind the curving section (27b),
   an encoder (55) for detecting a curve of the curving section of the endoscope insertion section by a pulley angle of the rotated pulley (52), and
   a tension sensor (34) for detecting tension applied to a wire (30) when the wire is pulled to cause the multiarticular manipulator (2) provided on the distal end side of the therapeutic device insertion section (18) to perform an articular operation,
   or is configured by combining these sensors with each other.

4. The therapeutic device system according to claim 3, **characterized in that**
   information on the curved state detected by the detection section (57) is one of
   curve information on the endoscope insertion section (27) based on a strain amount detected by the strain gauge,
   a drive amount for articulation-driving the articulation sections (42) on the basis of the pulley angle of the pulley (52) detected by the encoder (55),
   and a curve information on the therapeutic device insertion section (18) based on the wire tension detected by the tension sensor (34),
   or is information obtained by combining these pieces of information with each other.

5. The therapeutic device system according to claim 3, **characterized in that**
   the control parameter possessed by the control section (7) is one of
   a scale ratio of the manipulation-designated amount designated by the manipulation sections (3a and 3b) to a positional operation amount set to the therapeutic device (9),
   an operation ratio of a manipulation-designated angle designated by the manipulation sections (3a and 3b) to an angle to be formed at an articulation set to the therapeutic device (9),
   a sensitivity ratio by which a width of a dead band set to the therapeutic device (9) is changed with respect to a manipulation-designated amount designated by the manipulation sections (3a and 3b), and
   an individual input ratio for setting an operation condition to be set to the therapeutic device (9) with respect to a manipulation-designated amount designated by the manipulation sections (3a and 3b) for each operator who performs manipulation,
   or a parameter obtained by combining these parameters with each other.

6. The therapeutic device system according to claim 1, **characterized in that**
   in the manipulator (2), a plurality of curving pieces (41) are universal-joint-connected to each other by means of the articulation sections (42) such that a plurality of degrees of freedom are provided, and wires (43) are fixed to each of the curving pieces (41), and

the manipulator (2) is connected to a distal end of a therapeutic device insertion section (18) which is extended from the therapeutic device hole, and a therapeutic device (9) is provided at a distal end of the manipulator (2).

7. The therapeutic device system according to claim 6, **characterized in that**
the sensor section (57) includes
a strain gauge for detecting a curve of the endoscope insertion section (27) as a strain amount in order to acquire curved state information;
an encoder (55) for detecting a rotation amount of a pulley (52) provided on a shaft of a motor (53) serving as a drive source of the actuator; and
a tension sensor (34) for detecting tension applied to a wire (30) when the wire is pulled to cause the curving section provided on the distal end side of the therapeutic device insertion section (18) to perform a curving operation.

8. The therapeutic device system according to claim 6, **characterized in that**
the curved state information generated by the curved state information generation section (5) includes at least
curved state information on a strain amount of the endoscope insertion section (27) detected by using a strain gauge on the endoscope insertion section (27);
curved state information on the wire tension at the curving section provided on the distal end side of the endoscope insertion section (27); and
curved state information on the wire length selected at least one of a rotation amount of the pulley (52) detected by the encoder (55), a wire length of the wire pulled by the rotation amount of the pulley, and a wire length of the paid-out wire of the paid-out length.

9. The therapeutic device system according to claim 1, **characterized by** further comprising:

a therapeutic device (9) of an endoscope (20), or an active therapeutic device (9) to be attached after being passed through a therapeutic device hole of an overtube attached to the outside of the endoscope;
an endoscope curved state acquisition section (57) for acquiring a curved state of the endoscope (20);
an active therapeutic device drive section (32) for driving the active therapeutic device (9);
an active therapeutic device control section (7) for performing control while reflecting a control parameter set in advance in a drive control signal for controlling the active therapeutic device drive section (32), and changed by curved state information from the endoscope curved state acquisition section (57); and
an input section (3) by which an operator inputs an instruction to the active therapeutic device control section (7) for manipulating the therapeutic device (9) or the active therapeutic device (9).

10. The therapeutic device system according to claim 9, **characterized in that**
the endoscope (20) is an active endoscope which operates by means of electric power in accordance with the manipulation of an input section (29), and includes an endoscope drive section (54), an endoscope control section (56), and an endoscope input section (21).

11. The therapeutic device system according to claim 9, **characterized in that**
the curved state information of the endoscope is one of curve information on a curving tube section (27) of the endoscope (20), curve information on an insertion section (27b) other than the curving tube section (27) of the endoscope (20), and a rotation amount (rotation angle) of a pulley for pulling a wire (51) for curving the endoscope (20), or information obtained by combining these pieces of information with each other.

12. The therapeutic device system according to claim 9, **characterized in that**
the endoscope curved state acquisition section (57) is incorporated in the endoscope.

13. The therapeutic device system according to claim 9, **characterized in that**
the endoscope curved state acquisition section (5) is incorporated in the overtube.

14. The therapeutic device system according to claim 9, **characterized in that**
the endoscope curved state acquisition section (5) is incorporated in each of the endoscope (20) and the overtube, thereby configuring the system.

15. The therapeutic device system according to claim 10, **characterized in that**
the endoscope (20) is an endoscope which operates by means of electric power in accordance with an instruction given thereto by remote control of the operator.

**16.** The therapeutic device system according to claim 10, **characterized in that**
in the endoscope (20), the endoscope insertion section (27), and the endoscope input section (29) which is a manipulation section for the operator are configured to be attachable/detachable to/from each other.

**17.** The therapeutic device system according to claim 16, **characterized in that**
in the endoscope (20) configured to be attachable/detachable, a control parameter proper to each of a plurality of endoscope insertion sections which are exchangeably connected to the electric-powered curving manipulation section (29) is registered in advance in a table of a memory (37) provided in the active therapeutic device control section (7), and a control parameter corresponding to the endoscope insertion section (27) to be connected is read out and set at the time of connection.

**Patentansprüche**

**1.** Therapievorrichtungssystem umfassend:

einen Eingabeabschnitt (3) zum Erzeugen eines Manipulationssignals aus einem manipulationsbestimmten Wert, der durch Manipulationsabschnitte (3a und 3b) bestimmt wird;
eine Therapievorrichtung (9) oder einen Manipulator (2), welche(r) Gelenkabschnitte (42), einen Therapievorrichtungseinführabschnitt, der in eine Therapievorrichtungsöffnung eingeführt wird, die durch einen Endoskopeinführabschnitt (27) oder ein an der Außenseite des Endoskops angebrachtes Überrohr gebildet wird, und in welchem die Gelenkabschnitte (42) eine Gelenkoperation in Abhängigkeit eines Manipulationsbefehls des Eingabeabschnitts ausführen können;
einen Therapievorrichtungsantriebsabschnitt (6) zum Antreiben der Therapievorrichtung (9) und des Manipulators (2) in Abhängigkeit des Manipulationssignals;
**gekennzeichnet durch**
einen Detektionsabschnitt (57) zum Ermitteln eines gekrümmten Zustands des Endoskopeinführabschnitts (27) zu jedem Zeitpunkt; und
einen Steuerungsabschnitt (7), der mit einem im Vorraus gesetzten Steuerungsparameter versehen ist, zum Steuern der Gelenkoperation der Gelenkabschnitte **durch** Ändern der Steuerungsparameter zu jedem Zeitpunkt auf Grundlage eines Ermittlungsergebnisses des Detektionsabschnitts (57), und um den Therapievorrichtungsantriebsabschnitt (6) zu veranlassen, dass das Manipulationssignal den geänderten Steuerungsparameter reflektiert.

**2.** Therapievorrichtungssystem nach Anspruch 1, **dadurch gekennzeichnet, dass** es, um die Gelenkabschnitte (42) des Therapievorrichtungseinführabschnitts (27) zu veranlassen, eine Gelenkoperation durchzuführen, weiter umfasst:

Drähte (43), die mit den Gelenkabschnitten (42) verbunden sind;
Rollen (31) zum Durchführen eines Zusammenziehens der Drähte (30) durch eine Drehung; und
einen Rollenantriebsabschnitt (33) zum Drehen der Rollen (31).

**3.** Therapievorrichtungssystem nach Anspruch 1, **dadurch gekennzeichnet, dass** der Detektionsabschnitt (57) gebildet wird aus

einem Dehnmessstreifen zum Ermitteln einer Krümmung eines an einem distalen Endabschnitt des Endoskopeinführabschnitts gelegenen Krümmungsabschnitt (27b), und wobei der Einführabschnitt (27) hinter dem Krümmungsabschnitt (27b) angeordnet ist, oder
einem Encoder (55) zum Ermitteln einer Krümmung des Krümmungsabschnitts des Endoskopeinführabschnitts über einen Rollenwinkel der gedrehten Rolle (52), oder
einem Tensionssensor (34) zum Ermitteln der Zugbelastung an einem Draht (30), wenn der Draht gezogen wird, um den an einer distalen Endseite des Therapievorrichtungseinführabschnitts (18) gelegenen Viel-Gelenk-Manipulator (2) dazu zu veranlassen, eine Gelenkoperation durchzuführen, oder ist durch eine Kombination dieser Sensoren miteinander ausgebildet.

**4.** Therapievorrichtungssystem nach Anspruch 3, **dadurch gekennzeichnet, dass** Informationen über den gekrümmten Zustand, die von dem Detektionsabschnitt (57) ermittelt werden, umfassen:

Krümmungsinformationen an dem Endoskopeinführabschnitt (27) aufgrund eines Dehnungswertes, der durch den Dehnmessstreifen ermittelt wurde, oder
einen Antriebswert zum Antreiben der Gelenke der Gelenkabschnitte (42) aufgrund des Rollenwinkels der Rolle (52), der von dem Encoder (55) ermittelt wurde, oder
einer Krümmungsinformation an dem Therapievorrichtungseinführabschnitt (18) aufgrund der Zugbelastung an dem Draht, die von dem Tensionssensor (34) ermittelt wurde,
oder ist eine Information, die durch eine Kombination dieser Informationsteile miteinander erhalten wird.

5. Therapievorrichtungssystem nach Anspruch 3, **dadurch gekennzeichnet, dass** der Steuerungsparameter, über den der Steuerungsabschnitt (7) verfügt, umfasst:

   eine Verhältnisskala des manipulationsbestimmten Wertes, der durch die Manipulationsabschnitte (3a und 3b) auf einen für die Therapievorrichtung (9) gesetzten Positionsoperationswert bestimmt wird, oder
   ein Operationsverhältnis des manipulationsbestimmten Winkels, der durch die Manipulationsabschnitte (3a und 3b) auf einen bei einer Gelenkgruppe zu bildenden Winkel für die Therapievorrichtung (9) bestimmt wird, oder
   ein Empfindlichkeitsverhältnis, bei welchem eine für die Therapievorrichtung (9) gesetzte Unempfindlichkeitsbreite hinsichtlich eines manipulationsbestimmten Werts, der durch die Manipulationsabschnitte (3a und 3b) bestimmt wird, geändert wird; oder
   ein individuelles Eingabeverhältnis zum Einstellen eines für die Therapievorrichtung (9) hinsichtlich eines manipulationsbestimmten Wertes einzustellenden Operationszustands, der durch die Manipulationsabschnitte (3a und 3b) für jeden Operator, der die Manipulation durchführt, bestimmt wird,

   oder einen Parameter, der durch eine Kombination dieser Parameter miteinander erhalten wird.

6. Therapievorrichtungssystem nach Anspruch 1, **dadurch gekennzeichnet, dass**
   in dem Manipulator (2) eine Vielzahl von Krümmungsteilen (41) miteinander universell über Gelenke mittels der Gelenkabschnitte (42) verbunden sind, so dass eine Vielzahl von Freiheitsgraden bereitgestellt werden, und dass Drähte (43) mit jedem der Krümmungsteile (41) verbunden sind, und
   der Manipulator (2) mit einem distalen Ende des Therapievorrichtungseinführabschnitts (18) verbunden ist, welcher sich von der Therapievorrichtungsöffnung erstreckt, und dass eine Therapievorrichtung (9) an einem distalen Ende des Manipulators (2) vorgesehen ist.

7. Therapievorrichtungssystem nach Anspruch 6, **dadurch gekennzeichnet, dass** der Sensorabschnitt (57) umfasst:

   einen Dehnmessstreifen zum Ermitteln einer Krümmung des Endoskopeinführabschnitts (27) als ein Dehnungswert um Krümmungszustandsinformationen zu erhalten;
   einen Encoder (55) zum Ermitteln eines Drehwertes einer Rolle (52), die an einer Welle eines Motors (53), der als Antriebsquelle des Aktuators dient, angeordnet ist, und
   einen Tensionssensor (34) zum Ermitteln einer an einem Draht (30) vorherrschenden Zugbelastung, wenn der Draht auf Zug belastet wird, um den an der distalen Endseite des Therapievorrichtungseinführabschnitts (18) gelegenen Krümmungsabschnitt zu veranlassen, eine Krümmungsoperation durchzuführen.

8. Therapievorrichtungssystem nach Anspruch 6, **dadurch gekennzeichnet, dass** die Krümmungszustandsinformation, die von dem Krümmungszustandsinformationserzeugungsabschnitt (5) gebildet wird, aufweist:

   Krümmungszustandsinformationen an einem Dehnmesswert des Endoskopeinführabschnitts (27), die unter Verwendung eines Dehnmessstreifens an dem Endoskopeinführabschnitt (27) erfasst werden; oder
   Krümmungszustandsinformationen an der an dem Draht vorherrschenden Zugbelastung bei dem Krümmungsabschnitt, der an der distalen Endseite des Endoskopeinführabschnitts (27) vorgesehen ist; oder
   Krümmungszustandsinformationen an der Drahtlänge, die aus einem durch den Encoder (55) ermittelten Drehwert der Rolle (52), und/oder aus einer um den Drehbetrag der Rolle gezogenen Drahtlänge des Drahts, und/ oder aus einer Drahtlänge des abgerollten Drahts von der Abrolllänge ausgewählt wird.

9. Therapievorrichtungssystem nach Anspruch 1, **dadurch gekennzeichnet, dass** es weiter umfasst:

   eine Therapievorrichtung (9) eines Endoskops (20), oder eine aktive Therapievorrichtung (9), die, nachdem sie durch eine Therapievorrichtungsöffnung eines Überrohrs, welches an der Außenseite des Endoskops angebracht ist, geführt wurde, angebracht wird;
   einen Endoskopkrümmungszustand-Aufnahmeabschnitt (57), zum Aufnehmen eines Krümmungszustands des Endoskops (20);

einen aktive-Therapievorrichtungsantriebsabschnitt (32) zum Antreiben der aktiven Therapievorrichtung (9);

einen aktive-Therapievorrichtungssteuerungsabschnitt (7) zum Durchführen einer Steuerung, während dem Reflektieren eines Steuerungsparameters, der im Vorraus in einem Antriebssteuerungssignal zum Steuern des aktive-Therapievorrichtungsantriebsabschnitts (32) eingestellt wird, und durch Krümmungszustandsinformationen von dem Endoskopkrümmungszustand-Aufnahmeabschnitt (57) geändert wird; und

einen Eingabeabschnitt (3), bei welchem ein Operator einen Befehl für den aktive-Therapievorrichtungssteuerungsabschnitt (7) zum Manipulieren der Therapievorrichtung (9) oder der aktiven Therapievorrichtung (9) eingibt.

10. Therapievorrichtungssystem nach Anspruch 9, **dadurch gekennzeichnet, dass** das Endoskop (20) ein aktives Endoskop ist, welches mittels elektrischem Strom in Verbindung mit der Manipulation eines Eingabeabschnitts (29) arbeitet, und einen Endoskopantriebsabschnitt (54), einen Endoskopsteuerungsabschnitt (56) und einen Endoskopeingabeabschnitt (21) aufweist.

11. Therapievorrichtungssystem nach Anspruch 9, **dadurch gekennzeichnet, dass** die Krümmungszustandsinformation des Endoskops eine Krümmungsinformation an einem Krümmungsröhrenabschnitt (27) des Endoskops (20), und/oder eine Krümmungsinformation an einem Einführabschnitt (27b), welcher ein anderer als der Krümmungsabschnitt (27) des Endoskops (20) ist, und/oder ein Drehwert (Drehwinkel) einer Rolle zum Ziehen eines Drahtes (51) zum Krümmen des Endoskops (20) oder eine Information umfasst, die aus einer Kombination dieser Informationsteile miteinander erhalten wird.

12. Therapievorrichtungssystem nach Anspruch 9, **dadurch gekennzeichnet, dass** der Endoskopkrümmungszustand-Aufnahmeabschnitt (57) in dem Endoskop enthalten ist.

13. Therapievorrichtungssystem nach Anspruch 9, **dadurch gekennzeichnet, dass** der Endoskopkrümmungszustand-Aufnahmeabschnitt (5) in dem Überrohr enthalten ist.

14. Therapievorrichtungssystem nach Anspruch 9, **dadurch gekennzeichnet, dass** der Endoskopkrümmungszustand-Aufnahmeabschnitt (5) sowohl in dem Endoskop (20) als auch in dem Überrohr enthalten ist, um dabei das System zu konfigurieren.

15. Therapievorrichtungssystem nach Anspruch 10, **dadurch gekennzeichnet, dass** das Endoskop (20) ein Endoskop ist, welches mittels elektrischem Strom in Verbindung mit einem Befehl arbeitet, der über eine Fernsteuerung des Operators erzeugt wird.

16. Therapievorrichtungssystem nach Anspruch 10, **dadurch gekennzeichnet, dass** in dem Endoskop (20), der Endoskopeinführabschnitt (27) und der Endoskopeingabeabschnitt (29), welcher ein Manipulationsabschnitt für den Operator ist, so ausgestaltet sind, dass sie zusammengefügt und voneinander getrennt werden können.

17. Therapievorrichtungssystem nach Anspruch 16, **dadurch gekennzeichnet, dass** in dem zusammenfügbar und trennbar ausgebildeten Endoskop (20) ein Steuerungsparameter, der geeignet für jede von einer Vielzahl von Endoskopeinführabschnitten ist, welche austauschbar mit dem elektrisch betriebenen Krümmungsmanipulationsabschnitt (29) verbunden sind, im Vorraus in einer Tabelle eines in dem aktive-Therapievorrichtungsteuerungsabschnitt (7) vorgesehenen Speichers (37) aufgelistet wird, und ein dem zu verbindenden Endoskopeinführabschnitt (27) entsprechender Steuerungsparameter ausgelesen, und zum Zeitpunkt der Verbindung gesetzt wird.

**Revendications**

1. Système de dispositif thérapeutique comprenant :

une section d'entrée (3) pour générer un signal de manipulation à partir d'une quantité désignée de manipulation désignée par des sections de manipulation (3a et 3b) ;

l'un quelconque d'un dispositif thérapeutique (9) et d'un manipulateur (2) qui inclut des sections d'articulation (42), une section d'insertion de dispositif thérapeutique desquelles est insérée dans un trou de dispositif thérapeutique formé à travers l'un quelconque d'une section d'insertion d'endoscope (27) et d'un sur-tube fixé à l'extérieur d'un endoscope, et dans lesquels les sections d'articulation (42) peuvent exécuter une opération d'articulation conformément à une instruction de manipulation de la section d'entrée ;
une section d'entraînement de dispositif thérapeutique (6) pour entraîner le dispositif thérapeutique (9) et le manipulateur (2) conformément au signal de manipulation ;
**caractérisé par**
une section de détection (57) pour détecter un état de courbure de la section d'insertion d'endoscope (27) à tous moments ; et
une section de commande (7) prévue avec un paramètre de commande fixé au préalable, pour commander l'opération d'articulation des sections d'articulation en changeant le paramètre de commande à tous moments sur la base d'un résultat de détection de la section de détection (57), et en faisant en sorte que la section d'entraînement de dispositif thérapeutique (6) fasse que le signal de manipulation reflète le paramètre de commande changé.

2. Système de dispositif thérapeutique selon la revendication 1, **caractérisé en ce que** comprenant en outre, afin que les sections d'articulation (42) de la section d'insertion de dispositif thérapeutique (27) exécutent une opération d'articulation:

   des fils (43) raccordés aux sections d'articulation (42) ;
   des poulies (31) pour exécuter une traction des fils (30) par rotation ; et
   une section d'entraînement de poulies (33) pour mettre les poulies (31) en rotation.

3. Système de dispositif thérapeutique selon la revendication 1, **caractérisé en ce que**
   la section de détection (57) est constituée de l'un
   d'un extensomètre pour détecter une courbure d'une section de courbure (27b) prévue au niveau d'une section d'extrémité distale de la section d'insertion d'endoscope, et la section d'insertion (27) située derrière la section de courbure (27b),
   d'un encodeur (55) pour détecter une courbure de la section de courbure de la section d'insertion d'endoscope par un angle de poulie de la poulie (52) en rotation, et
   d'un détecteur de tension (34) pour détecter une tension appliquée à un fil (30) lorsque le fil est tiré pour faire en sorte que le manipulateur à articulations multiples (2) prévu sur le côté d'extrémité distale de la section d'insertion de dispositif thérapeutique (18) exécute une opération d'articulation,
   ou bien est configurée en combinant ces détecteurs les uns avec les autres.

4. Système de dispositif thérapeutique selon la revendication 3, **caractérisé en ce que**
   une information sur l'état de courbure détecté par la section de détection (57) est l'une
   d'une information de courbure sur la section d'insertion d'endoscope (27) sur la base d'une quantité de déformation détectée par l'extensomètre,
   d'une quantité d'entraînement pour un entraînement en articulation des sections d'articulation (42) sur la base de l'angle de poulie de la poulie (52) détecté par l'encodeur (55),
   et d'une information de courbure sur la section d'insertion de dispositif thérapeutique (18) sur la base de la tension de fil détectée par le détecteur de tension (34),
   ou bien est une information obtenue en combinant ces informations les unes avec les autres.

5. Système de dispositif thérapeutique selon la revendication 3, **caractérisé en ce que**
   le paramètre de commande possédé par la section de commande (7) est l'un d'un rapport d'échelle de la quantité désignée de manipulation désignée par les sections de manipulation (3a et 3b) sur une quantité d'opération positionnelle fixée pour le dispositif thérapeutique (9),
   d'un rapport opérationnel d'un angle désigné de manipulation désigné par les sections de manipulation (3a et 3b) sur un angle devant être formé au niveau d'une articulation fixé pour le dispositif thérapeutique (9),
   d'un rapport de sensibilité par lequel une largeur d'une bande morte fixée pour le dispositif thérapeutique (9) est changée en fonction d'une quantité désignée de manipulation désignée par les sections de manipulation (3a et 3b), et
   d'un rapport d'entrées individuelles pour fixer une condition opérationnelle devant être fixée pour le dispositif thérapeutique (9) en fonction d'une quantité désignée de manipulation désignée par les sections de manipulation (3a et 3b) pour chaque opérateur qui exécute une manipulation,
   ou bien un paramètre obtenu en combinant ces paramètres les uns avec les autres.

6. Système de dispositif thérapeutique selon la revendication 1, **caractérisé en ce que**
   dans le manipulateur (2), une pluralité de pièces de courbure (41) sont raccordées par un joint universel les unes aux autres au moyen des sections d'articulation (42) de telle sorte qu'une pluralité de degrés de liberté sont prévus, et des fils (43) sont fixés à chacune des pièces de courbure (41), et

le manipulateur (2) est raccordé à une extrémité distale d'une section d'insertion de dispositif thérapeutique (18) qui est étendue à partir du trou de dispositif thérapeutique, et un dispositif thérapeutique (9) est prévu au niveau d'une extrémité distale du manipulateur (2).

7. Système de dispositif thérapeutique selon la revendication 6, **caractérisé en ce que**

la section de détection (57) inclut

un extensomètre pour détecter une courbure de la section d'insertion d'endoscope (27) comme une quantité de déformation afin d'acquérir une information d'état de courbure ;

un encodeur (55) pour détecter une quantité de rotation d'une poulie (52) prévue sur un arbre d'un moteur (53) servant de source d'entraînement de l'actionneur ; et

un détecteur de tension (34) pour détecter une tension appliquée à un fil (30) lorsque le fil est tiré pour faire en sorte que la section de courbure prévue sur le côté d'extrémité distale de la section d'insertion de dispositif thérapeutique (18) exécute une opération d'articulation.

8. Système de dispositif thérapeutique selon la revendication 6, **caractérisé en ce que**

l'information d'état de courbure générée par la section de génération d'information d'état de courbure (5) inclut au moins

une information d'état de courbure sur une quantité de déformation de la section d'insertion d'endoscope (27) détectée en utilisant un extensomètre sur la section d'insertion d'endoscope (27) ;

une information d'état de courbure sur la tension de fil au niveau de la section de courbure prévue sur le côté d'extrémité distale de la section d'insertion d'endoscope (27) ; et

une information d'état de courbure sur la longueur de fil sélectionnée parmi au moins l'une d'une quantité de rotation de la poulie (52) détectée par l'encodeur (55), d'une longueur de fil du fil tiré par la quantité de rotation de la poulie, et d'une longueur de fil du fil déroulé de la longueur déroulée.

9. Système de dispositif thérapeutique selon la revendication 1, **caractérisé en ce que** comprenant en outre :

un dispositif thérapeutique (9) d'un endoscope (20), ou un dispositif thérapeutique actif (9) destiné à être fixé après avoir été passé à travers un trou de dispositif thérapeutique d'un sur-tube fixé sur l'extérieur de l'endoscope ;

une section d'acquisition d'état de courbure d'endoscope (57) pour acquérir un état de courbure de l'endoscope (20) ;

une section d'entraînement de dispositif thérapeutique actif (32) pour entraîner le dispositif thérapeutique actif (9) ;

une section de commande de dispositif thérapeutique actif (7) pour exécuter une commande tout en étant le reflet d'un paramètre de commande fixé au préalable dans un signal de commande d'entraînement pour commander la section d'entraînement de dispositif thérapeutique actif (32), et changé par l'information d'état de courbure provenant de la section d'acquisition d'état de courbure d'endoscope (57) ; et

une section d'entrée (3) par laquelle un opérateur entre une instruction dans la section de commande de dispositif thérapeutique actif (7) pour manipuler le dispositif thérapeutique (9) ou le dispositif thérapeutique actif (9).

10. Système de dispositif thérapeutique selon la revendication 9, **caractérisé en ce que**

l'endoscope (20) est un endoscope actif qui fonctionne au moyen d'une énergie électrique en fonction de la manipulation d'une section d'entrée (29), et inclut une section d'entraînement d'endoscope (54), une section de commande d'endoscope (56) et une section d'entrée d'endoscope (21).

11. Système de dispositif thérapeutique selon la revendication 9, **caractérisé en ce que**

l'information d'état de courbure de l'endoscope est l'une d'une information de courbure sur une section de tube de courbure (27) de l'endoscope (20), d'une information de courbure sur une section d'insertion (27b) autre que la section de tube de courbure (27) de l'endoscope (20), et d'une quantité de rotation (angle de rotation) d'un poulie pour tirer un fil (51) pour courber l'endoscope (20), ou d'une information obtenue en combinant ces informations les unes avec les autres.

12. Système de dispositif thérapeutique selon la revendication 9, **caractérisé en ce que**

la section d'acquisition d'état de courbure d'endoscope (57) est incorporée dans l'endoscope.

**13.** Système de dispositif thérapeutique selon la revendication 9, **caractérisé en ce que**
la section d'acquisition d'état de courbure d'endoscope (5) est incorporée dans le sur-tube.

**14.** Système de dispositif thérapeutique selon la revendication 9, **caractérisé en ce que**
la section d'acquisition d'état de courbure d'endoscope (5) est incorporée dans chacun de l'endoscope (20) et du sur-tube, configurant ainsi le système

**15.** Système de dispositif thérapeutique selon la revendication 10, **caractérisé en ce que**
l'endoscope (20) est un endoscope qui fonctionne au moyen d'une énergie électrique en fonction d'une instruction donnée à celui-ci par une commande à distance de l'opérateur.

**16.** Système de dispositif thérapeutique selon la revendication 10, **caractérisé en ce que**
dans l'endoscope (20), la section d'insertion d'endoscope (27), et la section d'entrée d'endoscope (29) qui est une section de manipulation pour l'opérateur sont configurées de manière à être attachables/détachables l'une à/de l'autre.

**17.** Système de dispositif thérapeutique selon la revendication 16, **caractérisé en ce que**
dans l'endoscope (20) configuré pour être attachable/détachable, un paramètre de commande propre à chacune d'une pluralité de sections d'insertion d'endoscope qui sont raccordées de façon échangeable à la section de manipulation de courbure (29) électrique est enregistré au préalable dans une table d'une mémoire (37) prévue dans la section de commande de dispositif thérapeutique actif (7), et un paramètre de commande correspondant à la section d'insertion d'endoscope (27) devant être raccordée est lu et fixé au moment du raccordement.

FIG. 1

F I G. 2A

F I G. 2B

EP 2 064 984 B1

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007111571 A1
- DE 3734979 A1
- WO 9933392 A1